# FASCICULE DE BREVET EUROPEEN

(11) **EP 3 013 948 B1**
(45) Date de publication et mention de la délivrance du brevet: **15.01.2020**
(21) Numéro de dépôt: 14739877.0
(22) Date de dépôt: 17.06.2014
(51) Int. Cl.: C12N 9/26

(54) **PROCEDE D'EXTRACTION DE BETA-AMYLASES A PARTIR D'UNE FRACTION SOLUBLE DE PLANTE AMIDONNIERE ET EN PRESENCE D'UNE PECTINASE**
VERFAHREN ZUR EXTRAKTION VON BETA-AMYLASEN AUS EINER LÖSLICHEN FRAKTION EINER STÄRKEPFLANZE IN GEGENWART EINER PECTINASE
METHOD FOR EXTRACTING BETA-AMYLASES FROM A SOLUBLE FRACTION OF A STARCH PLANT AND IN THE PRESENCE OF A PECTINASE

(30) Priorité: 24.06.2013 FR 1356022
(43) Date de publication de la demande: 04.05.2016
(73) Titulaire: Roquette Frères, 62136 Lestrem (FR)
(72) Inventeur: LECOCQ, Aline, 59110 La Madeleine (FR); COURBOIS, Vincent, F-62400 Bethune (FR); DUFLOT, Pierrick, F-62136 La Couture (FR)
(74) Mandataire: Plasseraud IP
(86) Numéro de dépôt international: PCT/FR2014/051490
(87) Numéro de publication internationale: WO 2014/207348

(56) Documents cités:
- EP-A2- 1 134 288
- FR-A1- 2 943 686
- YOUSSEF EL RAYESS ET AL: "Analysis of membrane fouling during cross-flow microfiltration of wine", INNOVATIVE FOOD SCIENCE & EMERGING TECHNOLOGIES, vol. 16, 1 octobre 2012 (2012-10-01), pages 398-408, XP055103408, ISSN: 1466-8564, DOI: 10.1016/j.ifset.2012.09.002
- J N Bacteriol ET AL: , 1 janvier 1988 (1988-01-01), page 5848, XP055103221, Extrait de l'Internet: URL:http://jb.asm.org/content/170/12/5848. full.pdf [extrait le 2014-02-19]
- D.R KASHYAP ET AL: "Applications of pectinases in the commercial sector: a review", BIORESOURCE TECHNOLOGY, vol. 77, no. 3, 10 août 2000 (2000-08-10), pages 215-227, XP055103353, ISSN: 0960-8524, DOI: 10.1016/S0960-8524(00)00118-8
- EKONG U. OKON ET AL: "Partial purification and properties of .beta.-amylase isolated from Sorghum bicolor (L.) Moench", JOURNAL OF AGRICULTURAL AND FOOD CHEMISTRY, vol. 32, no. 1, 1 janvier 1984 (1984-01-01), pages 11-14, XP055103212, ISSN: 0021-8561, DOI: 10.1021/jf00121a003
- DE BRUIJN J ET AL: "Influence of crossflow ultrafiltration on membrane fouling and apple juice quality", DESALINATION, ELSEVIER, AMSTERDAM, NL, vol. 148, no. 1-3, 10 septembre 2002 (2002-09-10), pages 131-136, XP004386584, ISSN: 0011-9164, DOI: 10.1016/S0011-9164(02)00666-5
- Anonymous: "Protoplast preparation (from plant tissue)", , 1 décembre 2006 (2006-12-01), XP055133165, Extrait de l'Internet: URL:http://www.ivaan.com/protocols/128.htm l [extrait le 2014-08-05]

## Description

La présente invention est relative à un procédé amélioré d'extraction de β-amylases à partir d'une fraction soluble de plante amidonnière, ledit procédé comprenant une étape de clarification par microfiltration et une étape de concentration / purification par ultrafiltration. Ce procédé fait appel à une pectinase au cours de l'étape d'ultrafiltration, ce qui améliore le rendement du procédé global ; de manière plus détaillée, on augmente ainsi la richesse en β-amylase du rétentat d'ultrafiltration, et on en diminue la viscosité, ce qui limite les phénomènes de montée en pression et de colmatage des membranes.

Les β-amylases sont des exo-hydrolases qui libèrent des unités maltose à partir des β-extrémités non réductrices des polymères ou oligomères de glucose liés en α 1→4, la réaction s'arrêtant au premier point de branchement α 1→6 rencontré. Composants majoritaires du « pouvoir diastatique » (correspondant aux activités combinées des α-amylases, β-amylases, α-glucosidases et enzymes débranchantes) durant le maltage (germination artificielle des graines de céréales), les activités β-amylasiques isolées de ce cocktail enzymatique sont essentielles pour la production du maltose ou d'autres sucres fermentescibles générés à partir d'amidon.

L'activité saccharifiante des seules β-amylases est donc exploitée dans bon nombre d'applications : en panification, en malterie, comme additif alimentaire, voire même comme agent digestif, pour la production d'édulcorants, en pharmacie pour la production de vaccins, et enfin pour la production de maltose et de sirops enrichis en maltose (précurseur du maltitol et des sirops de maltitol).

Les procédés de fabrication de β-amylases sont nombreux. On sait ainsi que les graines d'orge, de seigle ou de blé non germées sont autant de matériels biologiques de choix pour la préparation commerciale, à grande échelle, de β -amylases. Il est par ailleurs connu de l'homme du métier que la moitié des β-amylases extractibles des graines non germées de l'orge, du blé ou du seigle peut être facilement obtenue sous la forme d'enzymes libres par extraction avec de l'eau et des solutions salines. L'autre moitié se présente en partie sous forme « liée » qui nécessite l'addition d'agents réducteurs ou des enzymes protéolytiques pour son extraction. Une autre fraction de β-amylases non directement extractible, dite « latente » a également été décrite : il faut des détergents pour l'extraire des graines de céréales. Par ailleurs, les procédés d'extraction de la β-amylase décrits dans l'état de la technique sont adaptés en fonction de l'application visée.

A cet égard, la Demanderesse a récemment mis au point et protégé dans la Demande EP 2 414 379 un procédé original de production de β-amylases, en ce sens qu'il repose sur une matière première de départ jusqu'alors peu valorisée : les « fractions solubles ». Ces dernières étaient auparavant utilisées de manière exclusive comme source d'azote en fermentation et en tant qu'aliment nutritif pour le bétail une fois lesdites fractions enrichies en fibres.

Ces fractions solubles sont produites au cours de l'extraction par voie humide des composants des plantes amidonnières, comme notamment le maïs, la pomme de terre, la patate douce, le blé, le riz, le pois, la fève, la fèverole, le manioc, le sorgho, le konjac, le seigle, le sarrasin et l'orge. Les composants dits « nobles », produits au cours de l'extraction, sont notamment les amidons, les protéines ou encore les fibres. Les « fractions solubles » désignent par opposition des constituants « non nobles » : il s'agit des résidus liquides issus de ladite extraction, quand bien même de tels résidus peuvent encore contenir sous forme de traces des substances insolubles ainsi que des particules et des colloïdes divers et variés.

Le procédé objet de la Demande EP 2 414 379 repose sur la sélection initiale de la fraction soluble à traiter, sur une étape de clarification de celle-ci réalisée par microfiltration, puis sur une étape d'ultrafiltration pour concentrer le perméat de microfiltration contenant la β-amylase. A cette occasion, il a été démontré que la β-amylase obtenue était particulièrement bien adaptée à la préparation de sirops de maltose, au même titre qu'une β-amylase produite selon des techniques antérieures mais à partir de procédés plus complexes et plus onéreux.

Plus récemment, la Demanderesse a également protégé dans la Demande de Brevet Française n° 12 57680 non encore publiée, l'utilisation d'une protéase au cours de l'étape de clarification par microfiltration. On parvient ainsi à diminuer les problèmes de colmatage des membranes de microfiltration, ce qui revient à augmenter les temps de production entre chaque nettoyage / lavage desdites membranes.

Poursuivant ses travaux de développement sur cette technologie de production de β-amylases à partir de fractions solubles, la Demanderesse a été confrontée à un nouveau problème technique, relatif à l'étape d'ultrafiltration.

Au préalable, la Demanderesse rappelle que la première étape du procédé consiste à réaliser une étape de microfiltration des fractions solubles pour éliminer les substances insolubles, les colloïdes, et le matériel microbiologique en vue d'obtenir une composition limpide contenant de la β-amylase. Cette dernière composition se trouve donc être le perméat de microfiltration.

La deuxième étape du procédé est une étape d'ultrafiltration, visant à concentrer le perméat de microfiltration contenant la β-amylase, tout en le débarrassant d'éventuels sels résiduels contaminants, sucres et protéines. L'ultrafiltration est ainsi réalisée sur le perméat de la microfiltration de manière à obtenir un rétentat d'ultrafiltration contenant la β-amylase.

Or, il s'avère que le perméat de microfiltration est particulièrement riche en sucres complexes de type pentosane, ce qui entraîne 3 conséquences négatives :
- une viscosité importante au niveau du rétentat d'ultrafiltration ce qui le rend peu manipulable et crée une augmentation de la pression dans l'installation,
- une teneur en matière sèche élevée au niveau de ce rétentat, ce qui limite d'autant sa richesse en β-amylase,
- et le colmatage en surface, et interne, des membranes d'ultrafiltration, ce qui grève le rendement du procédé par la montée en pression rapide desdites membranes (il faut alors stopper le procédé pour nettoyer lesdites membranes ; la Demanderesse indique que la particularité des sucres complexes présents au niveau du rétentat est d'engendrer un colmatage interne des membranes, qui est beaucoup plus difficile à éliminer qu'un colmatage en surface).

Travaillant à la recherche de solutions techniques pour pallier ces problèmes, la Demanderesse est parvenue à démontrer que la mise en œuvre d'au moins une pectinase au cours de l'étape d'ultrafiltration, permettait de limiter très sensiblement ces effets indésirables. Aussi, l'objet de la présente invention consiste en un procédé d'extraction de β-amylases à partir d'une fraction soluble de plante amidonnière comprenant :
a) une étape de microfiltration d'une fraction soluble de plante amidonnière,
b) suivie d'une étape d'ultrafiltration du perméat de microfiltration,
ledit procédé étant caractérisé en ce que l'étape d'ultrafiltration est réalisée en présence d'au moins une pectinase.

Dans la présente Demande, le terme pectinases désigne des enzymes capables de décomposer les pectines qui sont des polymères de polysaccharide et qui sont l'un des constituants des parois cellulaires des plantes. Elles sont composées d'une chaîne principale d'acide uronique lié en 1-4. A ce titre, elles ne doivent pas être assimilées aux cellulases et aux hemicellulases auxquelles on les rattache par erreur : les cellulases sont des enzymes qui participent directement aux réactions de décomposition de la cellulose, (chaînes linéaires de molécules de D-glucose) alors que les hemicellulases parviennent à hydrolyser l'hemicellulose (polymères branchés de sucres en général, tels que le glucose, xylose, etc...).

Au sujet des cellulases et hemicellulases, on connaît le document EP 1 363 999 qui porte directement sur un procédé de fabrication de β-amylase par extraction en milieu aqueux en présence de cellulases, d'hémicellulases et de β-glucanases, ce dernier terme désignant des enzymes capables de décomposer les β-glucanes (polysaccharides entièrement constitués de D-glucose lié par des liaisons β 1-3, 1-4 et 1-6). Dans la présente Demande, il a notamment été démontré que les pectinases revendiquées conduisaient à de bien meilleurs résultats au niveau du module d'ultrafiltration, que les 3 enzymes citées plus haut dans le document EP 1 363 999.

Quant aux pectinases, elles sont encore peu utilisées pour le traitement ou la valorisation des matières amidonnières. On ne connaît à ce sujet que le document 1 591 019 portant sur le traitement de pommes de terre à l'aide de telles enzymes. Enfin, il est à noter que le document EP 0 552 728 décrit des pectines particulières, et dont on précise qu'elles n'ont aucune activité enzymatique vis-à-vis de l'amidon. Le document Analysis of membrane fouling during cross-flow microfilatration of wine d'El Rayess et al., Innovative Food Science & Emerging Technologies, Vol. 16, pages 398-408, décrit l'utilisation des pectinases pour hydrolyser les pectines du vin afin d'en faciliter la microfiltration.

L'objet de la présente invention consiste donc en un procédé d'extraction de β-amylases à partir d'une fraction soluble de plante amidonnière comprenant :
a) une étape de microfiltration d'une fraction soluble de plante amidonnière,
b) suivie d'une étape d'ultrafiltration du perméat de microfiltration,
ledit procédé étant caractérisé en ce que l'étape d'ultrafiltration est réalisée en présence d'au moins une pectinase.

Comme déjà expliqué, l'étape d'ultrafiltration vise tout d'abord à concentrer le perméat de microfiltration contenant la β-amylase, tout en le débarrassant d'éventuels sels résiduels contaminants, sucres et protéines. On introduit la pectinase dans le perméat de microfiltration avant de réaliser l'étape d'ultrafiltration. Avant de réaliser l'ultrafiltration, on laisse donc agir la pectinase au niveau du perméat de microfiltration : l'homme du métier saura adapter le temps de contact nécessaire à l'action de l'enzyme.

Typiquement, on laisse agir la pectinase de 30 minutes à 4 heures, préférentiellement de 30 minutes à 2 heures au plus et ce, à une température comprise entre 25°C et 60°C, préférentiellement entre 25°C et 50°C.

Des pectinases particulièrement adaptées à la mise en œuvre de la présente invention, sont les produits Rapidase™ ADEX D (pectinase ; DSM), Peclyve™ ESP (pectinase ; Lyven), ou Sumizyme™ ARS (pectinase et arabanase ; Takabio), sans pour autant que ces exemples ne soient limitatifs.

On préfèrera introduire une quantité de pectinase comprise entre 0,05 % et 1 % en volume en l'état de pectinase par rapport au volume total du perméat de microfiltration.

Plus particulièrement, la société Demanderesse recommande de réaliser l'ultrafiltration à l'aide de membranes présentant un seuil de coupure de 10 000 Da à 50 000 Da, de préférence un seuil de 30 000 Da. Les fractions solubles sont ultrafiltrées sur un module équipé de membranes polysulfonées d'un seuil de coupure de 30 000 Da en cassettes à l'échelle laboratoire et membranes spiralées polysulfonées d'un seuil de coupure de 30 000 Da à l'échelle pilote. L'enzyme se concentre alors dans le rétentat au fil du temps.

L'étape d'ultrafiltration peut être accompagnée d'une étape de dialyse du rétentat d'ultrafiltration de façon à diminuer la concentration en impuretés dans ledit rétentat.

En amont du procédé conforme à l'invention, il convient de choisir la fraction soluble de plantes amidonnières à traiter. Cette sélection s'opère notamment dans le groupe constitué des fractions solubles du blé, de la pomme de terre, du pois, de fève, de fèverole, du riz, de l'orge, du seigle, du sarrasin, de la patate douce et préférentiellement du blé et de l'orge.

La première étape du procédé conforme à l'invention consiste à réaliser une étape de microfiltration desdites fractions solubles. Cette étape a lieu avantageusement en présence d'au moins une protéase : dans ce cas, la protéase est mise en contact avec la fraction soluble de plante amidonnière à traiter (l'homme du métier saura adapter le temps de contact nécessaire à l'action de l'enzyme).

La protéase mise en œuvre dans la présente invention est choisie parmi les sérine protéases, les protéases à thiol, les aspartyl protéases et les métallo protéases, et est plus particulièrement choisie parmi les métallo protéases. De manière nominative, les protéases préférées dans la présente invention sont les produits commercialisés sous les dénominations : Sumizyme™ APL (Takabyo), Lypaine™ 6500 L (Lyven), Neutrase™ 0,8L (Novozymes), Brewlyve™ NP 900 (Lyven), Brewers Clarex™ (Brewers). On préfèrera utiliser une quantité de protéase comprise entre 0,01 % et 0,1 % en volume par rapport au volume de la fraction soluble de plante amidonnière à traiter.

L'étape de microfiltration du procédé conforme à l'invention est réalisée préférentiellement par microfiltration tangentielle membranaire. Plus particulièrement, la société Demanderesse recommande de réaliser la microfiltration tangentielle avec des membranes céramiques présentant une porosité de 0,1 µm à 1 µm.

De manière facultative, l'étape de microfiltration peut être précédée d'une étape de floculation des particules insolubles contenues dans la fraction soluble de plantes amidonnières, par toute technique connue par ailleurs de l'homme du métier.

Pour cette première étape de microfiltration, la Demanderesse recommande de travailler à un pH compris entre 4 et 5 et à une température comprise entre 40 °C et 50 °C.

Enfin, un deuxième objet de la présente invention consiste en l'utilisation d'au moins une pectinase dans un procédé d'extraction de β-amylases à partir d'une fraction soluble de plante amidonnière.

Les exemples qui suivent permettent de mieux appréhender l'invention, sans toutefois en limiter la portée.

### EXEMPLES

### Exemple 1

Cet exemple a pour objet de démontrer l'intérêt de mettre en œuvre des pectinases au cours de l'étape d'ultrafiltration, sur un procédé à l'échelle industrielle. Dans cet exemple, on réalise tout d'abord la microfiltration de fractions solubles de blé. Par la suite, on réalise l'étape d'ultrafiltration sans ajout d'enzyme (référence) ou avec une enzyme hors invention (cellulase, hemicellulase et β-glucanase) ou selon l'invention (pectinase).

On commence par prélever en amidonnerie de blé une fraction soluble à l'entrée de l'évaporateur des solubles, étape classiquement mise en œuvre pour fabriquer des produits destinés à l'alimentation du bétail, une fois concentrés. Ces produits sont commercialisés par la société Demanderesse sous le nom de Corami®. Ces fractions solubles présentent un pH compris entre 4 et 5 et une activité β-amytasique de l'ordre de 30 °DP / mL.

On réalise ici, sur un équipement à l'échelle pilote, la microfiltration de fractions solubles de blé. L'unité de microfiltration est équipée de membranes céramiques en oxyde de titane, dont le seuil de coupure est égal à 0,2 µm. Le débit de perméat est fixé à 12 L / h m². Le facteur de concentration volumique est égal à 1,5. La température et le pH du perméat sont respectivement égaux à 45°C et environ 4,5.

On ajoute dans la fraction soluble la protéase à tester, à une concentration fixée à 0,1 % en volume par rapport au volume total de ladite composition. On laisse au préalable agir cette protéase pendant 1 heure.

On obtient un perméat de microfiltration avec un degré DP de 25 °DP / ml après 1 heure de microfiltration, ce degré reflétant l'activité enzymatique de la solution contenant la β-amylase. La mesure de l'activité enzymatique est déterminée par l'activité diastasique. Cette dernière est exprimée en degré de pouvoir diastasique (°DP), défini comme la quantité d'enzyme contenue dans 0,1 ml d'une solution à 5 % d'un échantillon de préparation d'enzymes suffisante pour réduire 5 ml de liqueur de Fehling, quand ledit échantillon est placé dans 100 ml du substrat pendant 1 h à 20°C.

L'étape de microfiltration est suivie par une étape d'ultrafiltration, réalisée sur le perméat de microfiltration. Elle a pour principal objectif de concentrer ledit perméat et de le débarasser d'éventuels sels résiduels contaminants, sucres et protéines. Le pilote d'ultrafiltration est équipé de membranes organiques en polysulfone, ayant un seuil de coupure 25 KDa (membranes Alfa Laval).

Dans le cadre de la référence (essai n° 1), on réalise l'ultrafiltration du perméat sans rien lui ajouter.
Dans le cadre d'un essai comparatif, on ajoute au perméat le produit Optiflow™ RC 2.0 (Genencor) qui est une préparation enzymatique à base de cellulases, hemicellulase et β-glucanase (essai n°2)
Dans le cas de l'invention, on ajoute une pectinase qui est la Rapidase™ Adex D commercialisée par DSM (essai n° 3) et la Peclyve™ ESP commercialisée par Lyven (essai n°4).

Pour l'essai comparatif et l'invention, on ajoute dans le perméat de microfiltration l'enzyme à tester à une concentration de 0,1 % en volume par rapport au volume du produit à filtrer. On laisse au préalable agir cette enzyme pendant 1 heure à 35°C, la température de filtration est ensuite fixée à 25°C pour limiter au maximum les développements bactériologiques et préserver l'activité enzymatique.

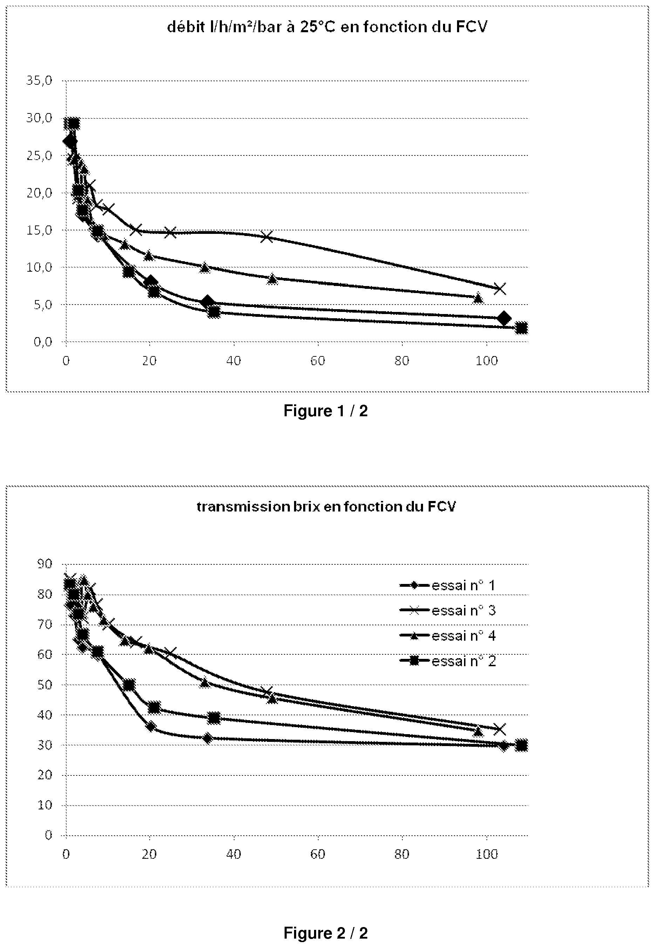

La Pression TransMembranaire (PTM) est fixée à 1 bar ; pour l'ensemble des essais on suit la baisse du débit de perméat en fonction du facteur de concentration volumique : ce flux décroit dans le temps au fur et à mesure que la membrane se colmate (figure 1). Plus le débit est important, meilleur est le rendement de filtration. On vérifie bien sur la figure 1 que les débits les plus importants sont obtenus pour les 2 pectinases.

Le % de transmission du brix permet quant à lui d'évaluer l'efficacité de l'hydrolyse des sucres complexes « indésirables », sucres en partie responsable de la viscosité du rétentat, de la montée en pression du système, et de la limitation de la montée en concentration de la préparation enzymatique (figure 2). Ce brix correspond à la fraction en sucres présents au niveau du perméat d'ultrafiltration. On constate sur la figure 2 que ce sont bien les 2 pectinases qui conduisent à un brix le plus important.

Enfin, le tableau 1 donne la teneur en matière (MS en %) sèche du rétentat d'ultrafiltration, de même que sa teneur en % en poids sec de protéines et de sucres.

La détermination de la teneur en protéines de ladite composition protéique conforme à l'invention est réalisée par la méthode de détermination de l'azote selon la méthode de DUMAS dans des échantillons dont la teneur présumée en azote est supérieure à 0,030 % (poids/poids), selon la norme NF V 18-120 - mars 1997. Cette teneur en protéines (N x 6,25) est exprimée en grammes pour 100 grammes de produit sec.

Les sucres totaux sont mesurés par chromatographie en phase gaz. Leur teneur est exprimée en % en poids sec de sucres totaux par rapport au poids sec total de la composition.

Ce sont bien les essais selon l'invention qui conduisent aux richesses les plus élevées en β-amylase et aux teneurs en sucres les plus faibles.

**Tableau 1**

| Essai n° | 1 | 3 | 4 | 2 |
|---|---|---|---|---|
| MS (%) | 26,4 | 14,7 | 17,1 | 28,3 |
| Protéines N x 6.25 (g / 100 g produit sec) | 43 | 88 | 73 | 48 |
| Sucres totaux (% sec) | 57% | 12% | 27% | 52% |

### Exemple 2

Cet exemple a pour objet de démontrer l'intérêt de mettre en œuvre des pectinases au cours de l'étape d'ultrafiltration, à travers des tests visant à déterminer la capacité de différentes enzymes à hydrolyser la fraction riche en sucres du perméat de microfiltration obtenu selon l'exemple 1.

Ces tests sont menés à l'échelle du laboratoire : ils permettent à travers de simples mesures du type HPLC, de discriminer rapidement les enzymes qui présentent un intérêt potentiel pour l'étape d'ultrafiltration réalisée à plus grande échelle.

De manière pratique, les enzymes à tester sont introduites dans des erlenmeyers dotés d'un barreau magnétique et contenant 100 ml du perméat de microfiltration, comme obtenu selon l'exemple 1.

La quantité d'enzyme ajoutée est de 1 ml. Dans le cas de mélange de plusieurs enzymes, le volume total des différentes enzymes est de 1 ml, réparti de manière égale pour chacune des enzymes. Le pH du substrat est inchangé et est compris entre 4 et 5. Les erlenmeyers sont placés dans un bain marie à 42°C sous agitation, le temps de contact est de 3 h. Avant l'analyse HPLC, les échantillons sont préalablement déminéralisés avec les résines AG4-X4 et AG50W-X8 (5 ml de chaque résine pour 10 ml d'échantillon), filtrés sur 0.45 µm et mis à brix 3 avec de l'eau.

Par la suite, on réalise une analyse de l'échantillon par chromatographie phase liquide (HPLC), cette étape mettant en œuvre un appareil HPLC de type Water 171 Plus Autosumpler équipé d'une colonne plomb Aminex HPX-87P. L'appareil est calibré à un débit de 0.4 µL/min et à une température de 85°C.

Cette technique permet de mesurer rapidement l'apparition des monomères et d'identifier ces derniers. Les résultats sont donnés en pourcentage de surface des pics, la répartition des surfaces sur le chromatogramme est représentative de la composition par rapport à la matière sèche ; on admet que toutes les espèces sont éluées et possèdent le même coefficient de réponse. L'ajout d'un étalon interne (galactitol) de concentration connue permet de calculer la concentration (en g/kg) de chacun des monomères présents dans l'échantillon.

On détermine alors pour chaque échantillon la teneur en glucose, xylose, galactose, arabinose, fructose et en sucres de degrés de polymérisation supérieurs, de même que la teneur en sucres libres totaux après une hydrolyse acide.

Les enzymes testées sont les suivantes :
- Celluclast™ 1.5 L (endoglucanase ; Novozymes)
- Ultraflow™ L (Betaglucanase et arabinofuranosidase ; Novozymes)
- Viscozyme™ L (betaglucanase ; Novozymes)
- Optiflow™ RC 2.0 (betaglucanase ; Genecor)
- Shearzyme™ 500L (xylanase ; Novozymes)
- Depol™ 740 L (Ferulic estérase Ferulic estérase ; Biocatalysts)
- Rapidase™ ADEX D (pectinase ; DSM)
- Peclyve™ ESP (pectinase ; Lyven)
- Sumizyme™ ARS (pectinase et arabanase ; Takabio)
- Viscoseb™ WWL (betaglucanase, xylanase et hemicellulose ; Seb)
- Optimash™ TGB (betaglucanase, xylanase et cellulose ; Genencor)
- Seb Grain™ B (pentosanase ; Seb)

Les résultats apparaissent dans les tableaux 2 et 3.

**Tableau 2**

| Résultats en g / kg | 0 | 1 | 2 | 3 | 4 | 5 |
|---|---|---|---|---|---|---|
| | Perméat MF | Celluclast 1,5 L | Ultraflow L | Optiflow RC2.0 | Shearzyme 500L | 1+2+Viscozyme L + Depol 740L |
| DP sup | 11,9 | 14,2 | 12,5 | 14,7 | 13,9 | 13,4 |
| Maltose | 17 | 21,1 | 21,2 | 18,8 | 20,4 | 13 |
| Glucose | 2,6 | 3,2 | 3,7 | 4,7 | 3,1 | 10,4 |
| xylose | 0,4 | 1,2 | 1,3 | 1,3 | 1,4 | 1,3 |
| galactose | 0,2 | 0,2 | 0,2 | 0,2 | 0,2 | 0,5 |
| arabinose | 0,3 | 0,4 | 1,5 | 0,7 | 0,2 | 1,4 |
| fructose | 1,3 | 1,5 | 1,4 | 1,4 | 1,4 | 1,7 |
| Total sucres libres | 4,8 | 6,4 | 8,1 | 8,2 | 6,3 | 15,4 |

**Tableau 3**

| Résultats en g / kg | 6 | 7 | 8 | 9 | 10 | 11 |
|---|---|---|---|---|---|---|
| | rapidase ADEX | peclyve ESP | sumizyme ARS | viscoseb WWL | optimash TGB | seb grain B |
| DP sup | 10,4 | 11,3 | 9,9 | 13,5 | 13,7 | 13,3 |
| Maltose | 7,3 | 8,9 | 4,7 | 17,7 | 18,5 | 23,6 |
| Glucose | 13,5 | 13,4 | 16,1 | 6,1 | 2,5 | 2,4 |
| xylose | 1,8 | 1,3 | 2,1 | 1 | 0,8 | 0,2 |
| galactose | 0,7 | 0,6 | 0,7 | 0,2 | 0,1 | 0 |
| arabinose | 1,6 | 1,3 | 1,4 | 0,5 | 0,1 | 0,1 |
| fructose | 4,9 | 1,9 | 6,2 | 1,7 | 1,2 | 1,1 |
| Total sucres libres | 22,4 | 18,5 | 26,4 | 9,6 | 4,6 | 3,8 |

En comparaison avec les autres enzymes les pectinases Rapidase™ Adex, Peclyve™ ESP et Sumizyme™ ARS présentent la plus grande efficacité pour l'hydrolyse de la fraction riche en sucres du perméat de microfiltration. Cet effet se traduit par une libération des sucres libres et une diminution des hauts degrés de polymérisation. Ces résultats ont pu être confirmés lors des essais de filtration sur pilote.

## Revendications

1. Procédé d'extraction de β-amylases à partir d'une fraction soluble de plante amidonnière comprenant :
a) une étape de microfiltration d'une fraction soluble de plante amidonnière,
b) suivie d'une étape d'ultrafiltration du perméat de microfiltration,
ledit procédé étant **caractérisé en ce que** l'étape d'ultrafiltration est réalisée en présence d'au moins une pectinase.

2. Procédé selon la revendication 1, **caractérisé en ce qu'**on introduit la pectinase dans le perméat de microfiltration avant de réaliser l'étape d'ultrafiltration.

3. Procédé selon l'une des revendications 1 ou 2, **caractérisé en ce qu'**il met en œuvre une pectinase à une concentration de 0,05% à 1 % en volume par rapport au volume de perméat de microfiltration.

4. Procédé selon l'une quelconque des revendications 1 à 3, **caractérisé en ce que** l'ultrafiltration met en œuvre au moins une membrane présentant un seuil de coupure de 10 000 Da à 50 000 Da, de préférence un seuil de 30 000 Da.

5. Procédé selon l'une quelconque des revendications 1 à 4, **caractérisé en ce que** l'étape d'ultrafiltration est accompagnée d'une étape de dialyse du rétentat d'ultrafiltration.

6. Procédé selon l'une quelconque des revendications 1 à 5, **caractérisé en ce que** l'étape de microfiltration a lieu en présence d'au moins une protéase, celle-ci étant mise en contact avec la fraction soluble de plante amidonnière.

7. Procédé selon l'une quelconque des revendications 1 à 6, **caractérisé en ce que** l'étape de microfiltration est réalisée préférentiellement par microfiltration tangentielle membranaire, préférentiellement avec des membranes céramiques présentant une porosité de 0,1 µm à 1 µm.

## Patentansprüche

1. Verfahren zur Extraktion von β-Amylasen aus einer löslichen Stärkepflanzenfraktion, umfassend:
a) einen Mikrofiltrationsschritt einer löslichen Stärkepflanzenfraktion,
b) gefolgt von einem Ultrafiltrationsschritt des Mikrofiltrationspermeats,
wobei das Verfahren **dadurch gekennzeichnet ist, dass** der Ultrafiltrationsschritt in Gegenwart von mindestens einer Pektinase durchgeführt wird.

2. Verfahren nach Anspruch 1, **dadurch gekennzeichnet, dass** die Pektinase vor Durchführung des Ultrafiltrationsschritts in das Mikrofiltrationspermeat eingebracht wird.

3. Verfahren nach einem der Ansprüche 1 oder 2, **dadurch gekennzeichnet, dass** es eine Pektinase in einer Konzentration von 0,05 Volumen-% bis 1 Volumen-% bezogen auf das Volumen des Mikrofiltrationspermeats umsetzt.

4. Verfahren nach einem der Ansprüche 1 bis 3, **dadurch gekennzeichnet, dass** die Ultrafiltration mindestens eine Membran mit einer Cut-off-Schwelle von 10.000 Da bis 50.000 Da, vorzugsweise einer Schwelle von 30.000 Da, einsetzt.

5. Verfahren nach einem der Ansprüche 1 bis 4, **dadurch gekennzeichnet, dass** der Ultrafiltrationsschritt von einem Dialyseschritt des Ultrafiltrationsretentats begleitet wird.

6. Verfahren nach einem der Ansprüche 1 bis 5, **dadurch gekennzeichnet, dass** der Mikrofiltrationsschritt in Gegenwart mindestens einer Protease stattfindet, wobei diese mit der löslichen Stärkepflanzenfraktion in Kontakt gebracht wird.

7. Verfahren nach einem der Ansprüche 1 bis 6, **dadurch gekennzeichnet, dass** der Mikrofiltrationsschritt vorzugsweise durch Tangential-Membranmikrofiltration durchgeführt wird, vorzugsweise mit Keramikmembranen mit einer Porosität von 0,1 µm bis 1 µm.

## Claims

1. Method for extracting β-amylases from a soluble fraction of starchy plant, comprising:
a) a step of microfiltration of a soluble fraction of starchy plant,
b) followed by a step of ultrafiltration of the microfiltration permeate,
said method being **characterised in that** said ultrafiltration step is performed in the presence of at least one pectinase.

2. Method according to claim 1, **characterised in that** the pectinase is introduced into the microfiltration permeate before performing the ultrafiltration step.

3. Method according to either claim 1 or claim 2, **characterised in that** it uses a pectinase at a concentration of 0.05% to 1% by volume with respect to the volume of microfiltration permeate.

4. Method according to any of claims 1 to 3, **characterised in that** the ultrafiltration uses at least one membrane having a cutoff threshold of 10,000 Da to 50,000 Da, preferably a threshold of 30,000 Da.

5. Method according to any of claims 1 to 4, **characterised in that** the ultrafiltration step is accompanied by a step of dialysis of the ultrafiltration residue.

6. Method according to any of claims 1 to 5, **characterised in that** the microfiltration step takes place in the presence of at least one protease, the latter being put in contact with the soluble fraction of starchy plant.

7. Method according to any of claims 1 to 6, **characterised in that** the microfiltration step is preferentially performed by a membrane tangential-flow microfiltration, preferentially with ceramic membranes having a porosity of 0.1 µm to 1 µm.
